# EUROPEAN PATENT APPLICATION

(11) **EP 2 168 600 A1**
(43) Date of publication of application: **31.03.2010**
(21) Application number: 08765247.5
(22) Date of filing: 06.06.2008
(51) Int. Cl.: A61K 45/00, A61K 31/275, A61K 31/336, A61K 31/495, A61K 31/496, A61K 31/55, A61P 9/00, A61P 43/00

(54) **THERAPEUTIC OR PROPHYLACTIC AGENT FOR CEREBRAL ANEURYSM**

(30) Priority: 08.06.2007 JP 2007152910
(71) Applicant: Nippon Chemiphar Co., Ltd., Chiyoda-ku Tokyo 101-0032 (JP)
(72) Inventor: HASHIMOTO, Nobuo, Kyoto-shi Kyoto 606-8501 (JP); KATAOKA, Hiroharu, Kyoto-shi Kyoto 606-8501 (JP); AOKI, Tomohiro, Kyoto-shi Kyoto 606-8501 (JP)
(74) Representative: Hiebl, Inge Elisabeth
(86) International application number: PCT/JP2008/060434
(87) International publication number: WO 2008/149971

(57) **Abstract**

[Problems] To provide a medicament which is effective for treatment and prophylaxis of cerebral aneurysms

[Means for solving the problems] The invention provides a medicament for treatment and prophylaxis of cerebral aneurysms, wherein the medicament contains a cysteine protease inhibitor as an active ingredient. The cysteine protease inhibitor preferably is an epoxysuccinamide derivative or its salt.

SEQUENCE LISTING

<110> Nippon Chemiphar Co., Ltd.

<120> Agent for treatment and prophylaxis of cerebral aneurysms

<130> 20891EP

<140> EP 08 765 247.5
<141> 2008-06-06

<150> JP20070152910
<151> 2007-06-08

<160> 10

<170> PatentIn version 3.1

<210> 1
<211> 23
<212> DNA
<213> Artificial Sequence

<220>
<223> forward primer for cathepsin B

<400> 1
aaatcaggcg tatacaagca tga 23

<210> 2
<211> 17
<212> DNA
<213> Artificial Sequence

<220>
<223> reverse primer for cathepsin B

<400> 2
gcccagaatg cggatgg 17

<210> 3
<211> 21
<212> DNA
<213> Artificial Sequence

<220>
<223> forward primer for cathepsin K

<400> 3
cccagactcc atcgactatc g 21

<210> 4
<211> 24
<212> DNA
<213> Artificial Sequence

<220>
<223> reverse primer for cathepsin K

<400>  4
ctgtaccctc tgcacttagc tgcc                                              24


<210>  5
<211>  20
<212>  DNA
<213>  Artificial Sequence

<220>
<223>  forward primer for cathepsin S

<400>  5
acgagcatcg actcagaagc                                                   20


<210>  6
<211>  20
<212>  DNA
<213>  Artificial Sequence

<220>
<223>  reverse primer for cathepsin S

<400>  6
tagccaacca cgagaacacc                                                   20


<210>  7
<211>  22
<212>  DNA
<213>  Artificial Sequence

<220>
<223>  forward primer for cathepsin C

<400>  7
ggattctcga ctcagagtat cc                                                22


<210>  8
<211>  22
<212>  DNA
<213>  Artificial Sequence

<220>
<223>  reverse primer for cathepsin C

<400>  8
gactgcacgt cttggacgga cg                                                22


<210>  9
<211>  21
<212>  DNA
<213>  Artificial Sequence

<220>
<223>  forward primer for beta-actin

<400>  9
aagcaatgct gtcaccttcc c                                                 21

<210>  10
<211>  22
<212>  DNA
<213>  Artificial Sequence

<220>
<223>  reverse primer for beta-actin

<400>  10
aagtccctca ccctcccaaa ag                                                22

## Description

### Field of the invention

The present invention relates to a medicament for treatment or prophylaxis of cerebral aneurysms. In more detail, the invention relates to a medicament for treatment or prophylaxis of progression or rupture of cerebral aneurysms.

### Background of the invention

Cerebral aneurysm is a major cause of catastrophic subarachnoid hemorrhage. A substantial number of patients die before arriving at a hospital. Even if patients survive the diseases, many of them suffer from serious sequela.

Despite the recent progress in surgery, the mortality of subarachnoid hemorrhage is still high. Craniotomy (clipping) and cerebroendovascular surgery have been conducted for treatment of cerebral aneurysms. Both of them are unsatisfied treatment because the patients' bodies suffer greatly from them.

The prevention of rupture of cerebral aneurysms is mandatory for public health. The main pathological features of cerebral aneurysms are disappearance of the internal elastic lamina and thinning of a tunica media, both of which are related to the degradation of extracellular matrix. Mechanism in progression of cerebral aneurysms and factors thereof remain to be elucidated.

The present inventors have studied matrix metalloproteases relating to the degradation of extracellular matrix, and reported a role of the matrix metalloproteases in the progression of cerebral aneurysms (cf., Non-patent document 1). Treatment with a selective inhibitor for matrix metalloproteases-2 and -9, however could not completely inhibit progression of cerebral aneurysms. The results suggest a presence of another factor different from the matrix metalloproteases.

Cathepsins consist of family members of lysosomal enzymes. Cathepsins digest unnecessary intracellular or endocytosed proteins. Recent molecular biological investigations revealed that some of cathepsins could function as proteases outside lysosomes. It has been known that cathepsins play crucial roles in pathological status, including bone diseases, inflammatory or autoimmune disease. However, the expression and role of cathepsins in cerebral aneurysms have not yet been examined.

Patent document 1 describes that nitrile derivatives having a function as a cathepsin K inhibitor are useful for the treatment or prophylaxis of various (about 20) diseases such as osteoporosis, osteoarthritis, rheumatoid arthritis, tumor metastasis, glomerulonephritis. Though examples of the diseases further include abdominal aortic aneurysm formation, Patent document 1 is silent with respect to cerebral aneurysms.
Patent document 1: Japanese Patent Publication No. 2003-519125
Non-patent document 1: Aoki Tomohiro et al., Macrophage-derived matrix metalloprotease-2 and -9 promote the progression of cerebral aneurysms in rats. Stroke. 2007;38:162-169

### Disclosure of the invention

### Problems to be solved by the invention

The object of the present invention is to provide a useful medicament for treatment or prophylaxis of cerebral aneurysms.

### Means to solve the problem

The present inventors have studied and discovered that expression of cathepsins B, K, and S in experimentally induced cerebral aneurysms. Each of the cathepsins B, K, and S is a cysteine protease. The present inventors have further studied to use a cysteine protease inhibitor for treatment or prophylaxis of cerebral aneurysms.

The present invention provides [1] a medicament for treatment or prophylaxis of cerebral aneurysms, wherein the medicament contains a cysteine protease inhibitor as an active ingredient.

[2] The medicament for treatment or prophylaxis is particularly effective in treatment or prophylaxis of progression or rupture of cerebral aneurysms.

[3] The cysteine protease inhibitor can inhibit one, two, or all of cathepsins B, K, and S.

[4] N-[1-[(cyanomethyl)carbamoyl]cyclohexyl]-4-(4-propylpyperazin-1-yl)benzamide, N-[(1S)-3-methyl-1-[[(4S,7R)-7-methyl-3-oxo-1-(pyridin-2-ylsulfonyl)hexahydro-1H-azepin-4-yl]carbamoyl]butyl]-1-benzofuran-2-carboxamide, (2R)-N-cyanomethyl-4-methyl-2-(4'-piperazin-1-yl-1,1'-biphenyl-3-yl)pentanamide, N-[3-[(2Z)-2-(3-methyl-1,3-thiazolin-2-ylidene)hydrazino]-2,3-dioxo-1-tetrahydro-2H-pyran-4-ylpropyl]cycloheptanecarboxamide, N-cyanomethyl-4-methyl-2-[2,2,2-trifluoro-1-(4'-methylsulfonyl-1,1'-biphenyl-4-yl)ethylamino]pentanamide, or monosodium (2S,3S)-3-[[(1S)-1-isobutoxymethyl-3-methylbutyl]carbamoyl]oxirane-2-carboxylate can be used as the cysteine protease inhibitor.

The invention also provides [5] a medicament for treatment or prophylaxis of cerebral aneurysms, wherein the medicament contains an epoxysuccinamide derivative having the formula (1) or its physiologically acceptable salt as an active ingredient.

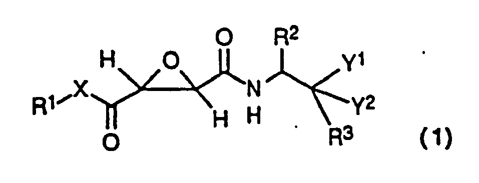

In the formula, R¹ represents a hydrogen atom, an alkyl group having 1 to 10 carbon atoms, an alkenyl group having 2 to 10 carbon atoms, an alkynyl group having 2 to 10 carbon atoms, an aryl group having 6 to 20 carbon atoms, an aralkyl group comprising an aryl group having 6 to 20 carbon atoms and an alkyl group having 1 to 6 carbon atoms, a heterocyclic group having 3 to 12 carbon atoms, or a heterocyclic-alkyl group comprising a heterocyclic group having 3 to 12 carbon atoms and an alkyl group having 1 to 6 carbon atoms.

R² represents an alkyl group having 1 to 10 carbon atoms, an alkenyl group having 2 to 10 carbon atoms, an alkynyl group having 2 to 10 carbon atoms, an aryl group having 6 to 20 carbon atoms, an aralkyl group comprising an aryl group having 6 to 20 carbon atoms and an alkyl group having 1 to 6 carbon atoms, a heterocyclic group having 3 to 12 carbon atoms, or a heterocyclic-alkyl group comprising a heterocyclic group having 3 to 12 carbon atoms and an alkyl group having 1 to 6 carbon atoms.

R³ represents a hydrogen atom, an alkyl group having 1 to 10 carbon atoms, an alkenyl group having 2 to 10 carbon atoms, an alkynyl group having 2 to 10 carbon atoms, an aryl group having 6 to 20 carbon atoms, an aralkyl group comprising an aryl group having 6 to 20 carbon atoms and an alkyl group having 1 to 6 carbon atoms, a heterocyclic group having 3 to 12 carbon atoms, or a heterocyclic-alkyl group comprising a heterocyclic group having 3 to 12 carbon atoms and an alkyl group having 1 to 6 carbon atoms.

X represents -O- or -NR⁴- in which R⁴ is a hydrogen atom, an alkyl group having 1 to 10 carbon atoms, an aryl group having 6 to 20 carbon atoms, an aralkyl group comprising an aryl group having 6 to 20 carbon atoms and an alkyl group having 1 to 6 carbon atoms, a heterocyclic group having 3 to 12 carbon atoms, or a heterocyclicalkyl group comprising a heterocyclic group having 3 to 12 carbon atoms and an alkyl group having 1 to 6 carbon atoms.

Y¹ represents OR⁵ in which R⁵ is a hydrogen atom, an alkyl group having 1 to 10 carbon atoms, an aryl group having 6 to 20 carbon atoms, an aralkyl group comprising an aryl group having 6 to 20 carbon atoms and an alkyl group having 1 to 6 carbon atoms, an acyl group having 2 to 20 carbon.atoms, a heterocyclic group having 3 to 12 carbon atoms, or a heterocyclic-alkyl group comprising a heterocyclic group having 3 to 12 carbon atoms and an alkyl group having 1 to 6 carbon atoms, SR⁶ in which R⁶ is a hydrogen atom, an alkyl group having 1 to 10 carbon atoms, an aryl group having 6 to 20 carbon atoms, an aralkyl group comprising an aryl group having 6 to 20 carbon atoms and an alkyl group having 1 to 6 carbon atoms, an acyl group having 2 to 20 carbon atoms, a heterocyclic group having 3 to 12 carbon atoms, or a heterocyclicalkyl group comprising a heterocyclic group having 3 to 12 carbon atoms and an alkyl group having 1 to 6 carbon atoms, or NR⁷R⁸ in which R⁷ is a hydrogen atom, an alkyl group having 1 to 10 carbon atoms, an aryl group having 6 to 20 carbon atoms, an aralkyl group comprising an aryl group having 6 to 20 carbon atoms and an alkyl group having 1 to 6 carbon atoms, an acyl group having 2 to 20 carbon atoms, a heterocyclic group having 3 to 12 carbon atoms, a heterocyclic-alkyl group comprising a heterocyclic group having 3 to 12 carbon atoms and an alkyl group having 1 to 6 carbon atoms, and R⁸ is a hydrogen atom, an alkyl group having 1 to 10 carbon atoms, an aryl group having 6 to 20 carbon atoms, an aralkyl group comprising an aryl group having 6 to 20 carbon atoms and an alkyl group having 1 to 6 carbon atoms, a heterocyclic group having 3 to 12 carbon atoms, or a heterocyclic-alkyl group comprising a heterocyclic group having 3 to 12 carbon atoms and an alkyl group having 1 to 6 carbon atoms.

Y² represents a hydrogen atom or an alkyl group having 1 to 10 carbon atoms. Alternatively Y¹ and Y² in combination with each other can form =O, =S, =N-R⁹ in which R⁹ is a hydrogen atom, an alkyl group having 1 to 10 carbon atoms, an aryl group having 6 to 20 carbon atoms, an aralkyl group comprising an aryl group having 6 to 20 carbon atoms and an alkyl group having 1 to 6 carbon atoms, a heterocyclic group having 3 to 12 carbon atoms, or a heterocyclic-alkyl group comprising a heterocyclic group having 3 to 12 carbon atoms and an alkyl group having 1 to 6 carbon atoms, or =N-OR¹⁰ in which R¹⁰ is a hydrogen atom, an alkyl group having 1 to 10 carbon atoms, an aryl group having 6 to 20 carbon atoms, an aralkyl group comprising an aryl group having 6 to 20 carbon atoms and an alkyl group having 1 to 6 carbon atoms, a heterocyclic group having 3 to 12 carbon atoms, or a heterocyclic-alkyl group comprising a heterocyclic group having 3 to 12 carbon atoms and an alkyl group having 1 to 6 carbon atoms.

Each of the alkyl groups for R⁵ to R¹⁰ can have one or more substituents selected from the group consisting of hydroxyl, amino, an alkylamino group having 1 to 6 carbon atoms, a dialkylamino group having 2 to 12 carbon atoms in total, an alkoxy group having 1 to 6 carbon atoms, carboxyl, an alkoxycarbonyl group having 2 to 7 carbon atoms, carbamoyl, an alkylaminocarbonyl group having 2 to 7 carbon atoms, a dialkylaminocarbonyl group having 3 to 13 carbon atoms in to tal, and guanidine, and each of the aryl groups and the heterocyclic groups for R¹ to R¹⁰ can have one or more substituents selected from the group consisting of an alkyl group having 1 to 6 carbon atoms, hydroxyl, amino, an alkylamino group having 1 to 6 carbon atoms, a dialkylamino group having 2 to 12 carbon atoms in total, an alkoxy group having 1 to 6 carbon atoms, a halogen atom, a haloalkyl group having 1 to 6 carbon atoms, cyano, nitro, carboxyl, an alkoxycarbonyl group having 2 to 7 carbon atoms, carbamoyl, an alkylaminocarbonyl group having 2 to 7 carbon atoms, a dialkylaminocarbonyl group having 3 to 13 carbon atoms in total, amidino, and guanidino.

The invention further provides [6] a medicament for treatment or prophylaxis having the formula (I), wherein R¹ represents a hydrogen atom, an alkyl group having 1 to 10 carbon atoms, an alkenyl group having 2 to 10 carbon atoms, an alkynyl group having 2 to 10 carbon atoms, an aryl group having 6 to 20 carbon atoms, an aralkyl group comprising an aryl group having 6 to 20 carbon atoms and an alkyl group having 1 to 6 carbon atoms, a heterocyclic group having 3 to 12 carbon atoms, or a heterocyclicalkyl group comprising a heterocyclic group having 3 to 12 carbon atoms and an alkyl group having 1 to 6 carbon atoms; R² represents an alkyl group having 1 to 10 carbon atoms, an alkenyl group having 2 to 10 carbon atoms, an alkynyl group having 2 to 10 carbon atoms, an aryl group having 6 to 20 carbon atoms, an aralkyl group comprising an aryl group having 6 to 20 carbon atoms and an alkyl group having 1 to 6 carbon atoms, a heterocyclic group having 3 to 12 carbon atoms, or a heterocyclic-alkyl group comprising a heterocyclic group having 3 to 12 carbon atoms and an alkyl group having 1 to 6 carbon atoms; R³ represents a hydrogen atom, an alkyl group having 1 to 10 carbon atoms, an alkenyl group having 2 to 10 carbon atoms, an alkynyl group having 2 to 10 carbon atoms, an aryl group having 6 to 20 carbon atoms, an aralkyl group comprising an aryl group having 6 to 20 carbon atoms and an alkyl group having 1 to 6 carbon atoms, a heterocyclic group having 3 to 12 carbon atoms, or a heterocyclicalkyl group comprising a heterocyclic group having 3 to 12 carbon atoms and an alkyl group having 1 to 6 carbon atoms; X represents -O- or -NR⁴- in which R⁴ is a hydrogen atom, an alkyl group having 1 to 10 carbon atoms, an aryl group having 6 to 20 carbon atoms, an aralkyl group comprising an aryl group having 6 to 20 carbon atoms and an alkyl group having 1 to 6 carbon atoms, a heterocyclic group having 3 to 12 carbon atoms, or a heterocyclicalkyl group comprising a heterocyclic group having 3 to 12 carbon atoms and an alkyl group having 1 to 6 carbon atoms; Y¹ represents hydroxyl, an alkoxy group having 1 to 6 carbon atoms, acetoxy, or an aralkyloxy group comprising an aryl group having 6 to 20 carbon atoms and an alkyl group having 1 to 6 carbon atoms; and Y² represents a hydrogen atom or an alkyl group having 1 to 10 carbon atoms; provided that each of the aryl groups and the heterocyclic groups for R¹ to R⁴ can have one or more substituents selected from the group consisting of an alkyl group having 1 to 6 carbon atoms, hydroxyl, amino, an alkylamino group having 1 to 6 carbon atoms, a dialkylamino group having 2 to 12 carbon atoms in total, an alkoxy group having 1 to 6 carbon atoms, a halogen atom, a haloalkyl group having 1 to 6 carbon atoms, cyano, nitro, carboxyl, an alkoxycarbonyl group having 2 to 7 carbon atoms, carbamoyl, an alkylaminocarbonyl group having 2 to 7 carbon atoms, a dialkylaminocarbonyl group having 3 to 13 carbon atoms in total, amidino, and guanidino.

The invention further provides [7] a medicament for treatment or prophylaxis of cerebral aneurysms, wherein the medicament contains an epoxysuccinamide derivative having the formula (1) or its physiologically acceptable salt as an active ingredient.

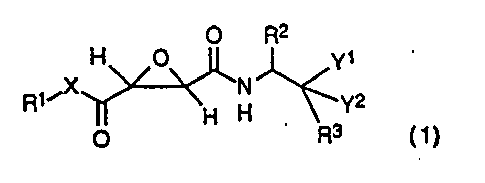

In the formula, R¹ represents a hydrogen atom, an alkyl group having 1 to 10 carbon atoms, an alkenyl group having 2 to 10 carbon atoms, an alkynyl group having 2 to 10 carbon atoms, or an aryl group having 6 to 20 carbon atoms, an aralkyl group comprising an aryl group having 6 to 20 carbon atoms and an alkyl group having 1 to 6 carbon atoms; R² represents isobutyl or isopropyl; R³ represents a hydrogen atom or an aryl group having 6 to 20 carbon atoms; X represents -O- or -NR⁴- in which R⁴ is a hydrogen atom, an alkyl group having 1 to 10 carbon atoms, an aryl group having 6 to 20 carbon atoms, an aralkyl group comprising an aryl group having 6 to 20 carbon atoms and an alkyl group having 1 to 6 carbon atoms, a heterocyclic group having 3 to 12 carbon atoms, or a heterocyclic-alkyl group comprising a heterocyclic group having 3 to 12 carbon atoms and an alkyl group having 1 to 6 carbon atoms; Y¹ represents OR⁵ in which R⁵ is a hydrogen atom, an alkyl group having 1 to 10 carbon atoms, an aryl group having 6 to 20 carbon atoms, an aralkyl group comprising an aryl group having 6 to 20 carbon atoms and an alkyl group having 1 to 6 carbon atoms, acetyl, benzoyl, a heterocyclic group having 3 to 12 carbon atoms, or a heterocyclic-alkyl group comprising a heterocyclic group having 3 to 12 carbon atoms and an alkyl group having 1 to 6 carbon atoms; Y² represents a hydrogen atom; provided that the alkyl group for R⁵ can have one or more substituents selected from the group consisting of hydroxyl, amino, an alkylamino group having 1 to 6 carbon atoms, a dialkylamino group having 2 to 12 carbon atoms in total, an alkoxy group having 1 to 6 carbon atoms, carboxyl, an alkoxycarbonyl group having 2 to 7 carbon atoms, -CONH₂, an alkylaminocarbonyl group having 2 to 7 carbon atoms, a dialkylaminocarbonyl group having 3 to 13 carbon atoms in total, and guanidino; each of the aryl groups for R¹, R³ and R⁵ can have one or more substituents selected from the group consisting of an alkyl group having 1 to 6 carbon atoms, hydroxyl, amino, an alkylamino group having 1 to 6 carbon atoms, a dialkylamino group having 2 to 12 carbon atoms in total, an alkoxy group having 1 to 6 carbon atoms, a halogen atom, a haloalkyl group having 1 to 6 carbon atoms, cyano, nitro, carboxyl, an alkoxycarbonyl group having 2 to 7 carbon atoms, - CONH₂, an alkylaminocarbonyl group having 2 to 7 carbon atoms, a dialkylaminocarbonyl group having 3 to 13 carbon atoms in total, amidino, and guanidine; the heterocyclic group for R⁵ can have one or more substituents selected from the group consisting of an alkyl group having 1 to 6 carbon atoms, hydroxyl, amino, an alkylamino group having 1 to 6 carbon atoms, a dialkylamino group having 2 to 12 carbon atoms in total, an alkoxy group having 1 to 6 carbon atoms, a halogen atom, a haloalkyl group having 1 to 6 carbon atoms, cyano, nitro, carboxyl, an alkoxycarbonyl group having 2 to 7 carbon atoms, -CONH₂, an alkylaminocarbonyl group having 2 to 7 carbon atoms, a dialkylaminocarbonyl group having 3 to 13 carbon atoms in total, amidino, and guanidine; and each of the heterocyclic groups for R⁴ and R⁵ is selected from the group consisting of pyridyl, pyrrolidinyl, piperidinyl, furyl, thienyl, piperazinyl, indolyl, and benzimidazolyl.

[8] The medicament for treatment or prophylaxis as defined in one of [5] to [7], wherein R¹ is a hydrogen atom or an alkyl group having 1 to 6 carbon atoms.

[9] The medicament for treatment or prophylaxis as defined in [5] or [6], wherein R² is an alkyl group having 1 to 6 carbon atoms, phenyl, or benzyl.

[10] The medicament for treatment or prophylaxis as defined in [5] or [6], wherein R³ is a hydrogen atom or an aryl group having 6 to 20 carbon atoms.

[11] The medicament for treatment or prophylaxis as defined in one of [5] to [7], wherein X is -O-.

[12] The medicament for treatment or prophylaxis as defined in [5], wherein Y¹ is hydroxyl, an alkoxy group having 1 to 6 carbon atoms, acetoxy, or an aralkyloxy group comprising an aryl group having 6 to 20 carbon atoms and an alkyl group having 1 to 6 carbon atoms.

[13] The medicament for treatment or prophylaxis as defined in [5], wherein R² is isobutyl or isopropyl, R³ is a hydrogen atom, Y¹ is OR⁵ whose R⁵ has the meaning defined in [5], and Y² is a hydrogen atom.

[14] The medicament for treatment or prophylaxis as defined in [5], wherein R² is isobutyl or isopropyl, R³ is an aryl group having 6 to 20 carbon atoms, Y¹ is OR⁵ whose R⁵ has the meaning defined in [5], and Y² is a hydrogen atom.

[15] The medicament for treatment or prophylaxis as defined in [5], wherein Y¹ and Y² in combination with each other forms =O.

[16] The medicament for treatment or prophylaxis as defined in one of [5] to [15], wherein the physiologically acceptable salt is an alkali metal salt.

The invention further provides [17] a medicament for treatment or prophylaxis of cerebral aneurysms, wherein the medicament contains an epoxysuccinamide derivative having the following formula or its physiologically acceptable salt as an active ingredient.

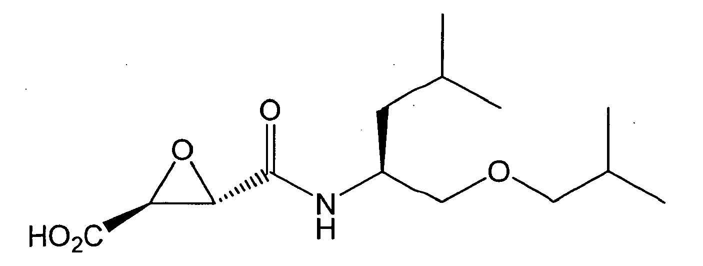

### Effect of the invention

According to study of the present inventors, it has been revealed that expression of cathepsins B, K, and S is induced in cerebral aneurysms, and expression of cystatin C, an endogenous inhibitor of cathepsin, is reduced.

In the late stage of aneurysm progression, mRNA of cathepsin B, K, and S is increased. While cystatin C is highly expressed in the normal cerebral arterial walls, its expression is reduced in cerebral aneurysms.

Each of the cathepsins B, K, and S is a cysteine protease. The present inventors have succeeded in inhibiting progression of cerebral aneurysms by using a cysteine protease. The results revealed that an imbalance between cathepsins and cystatin C in cerebral artery causes the progression of cerebral aneurysms, which further causes rupture of cerebral aneurysms.

The medicament containing a cysteine protease inhibitor as an active ingredient can effectively be used according to the present invention for treatment or prophylaxis of cerebral aneurysms, particularly treatment or prophylaxis of progression or rupture of cerebral aneurysms, which has not been realized according to prior art.

### The best mode of the invention

In the present specification, the term "cysteine protease" means a protease having SH group at the active center, which is the same as the general definition. Each of the cathepsins B, K, and S has been revealed to involve in formation of cerebral aneurysms, and each of them is a cysteine protease. Therefore, it is expected that widely and various kinds of cysteine protease inhibitors are effective for treatment or prophylaxis of cerebral aneurysms.

The cysteine protease inhibitors, particularly the inhibitors of the cathepsins B, K, and S, which are cysteine proteases, are described in WO97/21694, WO98/47887, WO01/049288, WO01/058886, WO01/070232, WO03/053331, WO03/075836, WO03/091202, W02004/108661, W02005/000800, W02005/066180, W02007/025774, WO2007/009715, and Japanese Patent Provisional Publication No. 11(1999)-263783. The cathepsins B, K, and S are also described in various non-patent documents such as T. Yasuma et al., J. Med. Chem. 1998, 41, 4301-4308; N. Katumuma et al., FEBS Lett., 1999, 458, 6-10; T. Otsuka et al., J. Antibiot., 1999, 52, 536-541; R.W. Marquis et al., J. Med. Chem. 2001, 44, 1380-1395; P.D. Greenspan et al., Bioorg., Med. Chem. Lett., 2003, 13, 4121-4124; J. Robichaud et al., J. Med. Chem. 2003, 46, 3709-3727; C.S. Li et al., Bioorg., Med. Chem. Lett., 2006, 16, 1985-1989.

Examples of the cysteine protease inhibitors include N-[1-[(cyanomethyl)carbamoyl]cyclohexyl]-4-(4-propylpyperazin-1-yl)benzamide, N-[(1S)-3-methyl-1-[[(4S,7R)-7-methyl-3-oxo-1-(pyridin-2-ylsulfonyl)hexahydro-1H-azepin-4-yl]carbamoyl]butyl]-1-benzofuran-2-carboxamide, (2R)-N-cyanomethyl-4-methyl-2-(4'-piperazin-1-yl-1,1'-biphenyl-3-yl)pentanamide, N-[3-[(2 Z)-2-(3-methyl-1,3-thiazolin-2-ylidene)hydrazino]-2,3-dioxo-1-tetrahydro-2H-pyran-4-ylpropyl]cycloheptanecarboxamide, and N-cyanomethyl-4-methyl-2-[2,2,2-trifluoro-1-(4'-methylsulfonyl-1,1'-biphenyl-4-yl)ethylamino]pentanamide.

The cysteine protease inhibitor preferably is an epoxysuccinamide derivative having the above-mentioned formula (1) or its physiologically acceptable salt. The epoxysuccinamide derivative having the above-mentioned formula (1) or its physiologically acceptable salt has been invented as an inhibitor of a cysteine protease such as cathepsins B and L for treatment or prophylaxis of bone diseases (cf., WO99/11640). The recent recearch has revealed that these compounds further inhibit cathepsin K.

The epoxysuccinamide derivative having the formula (1) is further described below in more detail.

In the formula (1), R¹ is a hydrogen atom, an alkyl group having 1 to 10 carbon atoms (preferably an alkyl group having 1 to 6 carbon atoms, optionally a linear, branched, or cyclic alkyl group, e.g., methyl, ethyl, n-propyl, isopropyl, n-butyl, isobutyl, sec-butyl, tert-butyl, pentyl, isopentyl, hexyl, and cyclohexyl), an alkenyl group having 2 to 10 carbon atoms (preferably an alkenyl group having 2 to 6 carbon atoms, optionally a linear, branched, or cyclic alkenyl group, e.g., allyl, 2-methyl-1-propenyl, and 2-cyclohexenyl), an alkynyl group having 2 to 10 carbon atoms (preferably an alkynyl group having 2 to 6 carbon atoms, optionally a linear or branched alkynyl group, e.g., 2-propynyl and 3-butynyl), an aryl group having 6 to 20 carbon atoms (e.g., phenyl and naphthyl), an aralkyl group comprising an aryl group having 6 to 20 carbon atoms and an alkyl group having 1 to 6 carbon atoms (e.g., benzyl, phenethyl and 3-phenylpropyl), a heterocyclic group having 3 to 12 carbon atoms (e.g., pyridyl, pyrrolidinyl, piperidinyl, furyl, and thienyl), or a heterocyclic-alkyl group comprising a heterocyclic group having 3 to 12 carbon atoms and an alkyl group having 1 to 6 carbon atoms (e.g., furfuryl, 2-thenyl, and 2-(3-pyridyl)ethyl).

R² is an alkyl group having 1 to 10 carbon atoms (preferably an alkyl group having 1 to 6 carbon atoms, optionally a linear, branched, or cyclic alkyl group, e.g., methyl, ethyl, n-propyl, isopropyl, n-butyl, isobutyl, sec-butyl, pentyl, isopentyl, hexyl, and cyclohexyl), an alkenyl group having 2 to 10 carbon atoms (preferably an alkenyl group having 2 to 6 carbon atoms, optionally a linear, a branched, or a cyclic alkenyl group, e.g., vinyl, 2-methyl-1-propenyl, and 2-cyclohexenyl), an alkynyl group having 2 to 10 carbon atoms (preferably an alkynyl group having 2 to 6 carbon atoms, optionally a linear or branched alkynyl group, e.g., 2-propynyl and 3-butynyl), an aryl group having 6 to 20 carbon atoms (e.g., phenyl and naphthyl), an aralkyl group comprising an aryl group having 6 to 20 carbon atoms and an alkyl group having 1 to 6 carbon atoms (e.g., benzyl, phenethyl and 3-phenylpropyl), a heterocyclic group having 3 to 12 carbon atoms (e.g., pyridyl, pyrrolidinyl, piperidinyl, furyl, and thienyl), or a heterocyclic-alkyl group comprising a heterocyclic group having 3 to 12 carbon atoms and an alkyl group having 1 to 6 carbon atoms (e.g., 3-indolylmethyl and 2-pyridylmethyl)

R³ is a hydrogen atom, an alkyl group having 1 to 10 carbon atoms (preferably an alkyl group having 1 to 6 carbon atoms, optionally a linear, branched, or cyclic alkyl group, e.g., methyl, ethyl, n-propyl, isopropyl, n-butyl, isobutyl, sec-butyl, pentyl, isopentyl and hexyl), an alkenyl group having 2 to 10 carbon atoms (preferably an alkenyl group having 2 to 6 carbon atoms, optionally a linear, a branched, or a cyclic alkenyl group, e.g., vinyl, 2-metlhyl-1-propenyl, and 2-cyclohexenyl), an alkynyl group having 2 to 10 carbon atoms (preferably an alkynyl group having 2 to 6 carbon atoms, optionally a linear or branched alkynyl group, e.g., 2-propynyl and 3-butynyl), an aryl group having 6 to 20 carbon atoms (e.g., phenyl and naphthyl), an aralkyl group comprising an aryl group having 6 to 20 carbon atoms and an alkyl group having 1 to 6 carbon atoms (e.g., benzyl, phenethyl and 3-phenylpropyl), a heterocyclic group having 3 to 12 carbon atoms (e.g., pyridyl, pyrrolidinyl, piperidinyl, furyl, and thienyl), or a heterocyclic-alkyl group comprising a heterocyclic group having 3 to 12 carbon atoms and an alkyl group having 1 to 6 carbon atoms (e.g., 3-indolylmethyl and 2-pyridylmethyl).

X is -O- or -NR⁴- (in which R⁴ is a hydrogen atom, an alkyl group having 1 to 10 carbon atoms, an aryl group having 6 to 20 carbon atoms, an aralkyl group comprising an aryl group having 6 to 20 carbon atoms and an alkyl group having 1 to 6 carbon atoms, a heterocyclic group having 3 to 12 carbon atoms, or a heterocyclic-alkyl group comprising a heterocyclic group having 3 to 12 carbon atoms and an alkyl group having 1 to 6 carbon atoms). Preferred carbon atom numbers for these alkyl, aryl, aralkyl and heterocyclic-alkyl groups and their concrete examples are those described above for R¹, R² and R³.

Y¹ is OR⁵ (in which R⁵ is a hydrogen atom, an alkyl group having 1 to 10 carbon atoms, an aryl group having 6 to 20 carbon atoms, an aralkyl group comprising an aryl group having 6 to 20 carbon atoms and an alkyl group having 1 to 6 carbon atoms, an acyl group having 2 to 20 carbon atoms, a heterocyclic group having 3 to 12 carbon atoms, or a heterocyclic-alkyl group comprising a heterocyclic group having 3 to 12 carbon atoms and an alkyl group having 1 to 6 carbon atoms), SR⁶ (in which R⁶ is a hydrogen atom, an alkyl group having 1 to 10 carbon atoms, an aryl group having 6 to 20 carbon atoms, an aralkyl group comprising an aryl group having 6 to 20 carbon atoms and an alkyl group having 1 to 6 carbon atoms, an acyl group having 2 to 20 carbon atoms, a heterocyclic group having 3 to 12 carbon atoms, or a heterocyclicalkyl group comprising a heterocyclic group having 3 to 12 carbon atoms and an alkyl group having 1 to 6 carbon atoms), or NR⁷R⁸ (in which R⁷ is a hydrogen atom, an alkyl group having 1 to 10 carbon atoms, an aryl group having 6 to 20 carbon atoms, an aralkyl group comprising an aryl group having 6 to 20 carbon atoms and an alkyl group having 1 to 6 carbon atoms, an acyl group having 2 to 20 carbon atoms, a heterocyclic group having 3 to 12 carbon atoms, a heterocyclic-alkyl group comprising a heterocyclic group having 3 to 12 carbon atoms and an alkyl group having 1 to 6 carbon atoms, and R⁸ is a hydrogen atom, an alkyl group having 1 to 10 carbon atoms, an aryl group having 6 to 20 carbon atoms, an aralkyl group comprising an aryl group having 6 to 20 carbon atoms and an alkyl group having 1 to 6 carbon atoms, a heterocyclic group having 3 to 12 carbon atoms, or a heterocyclic-alkyl group comprising a heterocyclic group having 3 to 12 carbon atoms and an alkyl group having 1 to 6 carbon atoms). Preferred carbon atom numbers for these alkyl, aryl, aralkyl and heterocyclic-alkyl groups and their concrete examples are those described above for R¹, R² and R³.

Y² is a hydrogen atom or an alkyl group having 1 to 10 carbon atoms (preferably an alkyl group having 1 to 6 carbon atoms, optionally a linear, branched, or cyclic alkyl group, e.g., methyl, ethyl, n-propyl, isopropyl, n-butyl, isobutyl, sec-butyl, pentyl, isopentyl and hexyl).

In the formula (1), Y¹ and Y² can form alternatively, in combination with each other, =O, =S, =N-R⁹ (in which R⁹ is a hydrogen atom, an alkyl group having 1 to 10 carbon atoms, an aryl group having 6 to 20 carbon atoms, an aralkyl group comprising an aryl group having 6 to 20 carbon atoms and an alkyl group having 1 to 6 carbon atoms, a heterocyclic group having 3 to 12 carbon atoms, or a heterocyclic-alkyl group comprising a heterocyclic group having 3 to 12 carbon atoms and an alkyl group having 1 to 6 carbon atoms), or =N-OR¹⁰ (in which R¹⁰ is a hydrogen atom, an alkyl group having 1 to 10 carbon atoms, an aryl group having 6 to 20 carbon atoms, an aralkyl group comprising an aryl group having 6 to 20 carbon atoms and an alkyl group having 1 to 6 carbon atoms, a heterocyclic group having 3 to 12 carbon atoms, or a heterocyclic-alkyl group comprising a heterocyclic group having 3 to 12 carbon atoms and an alkyl group having 1 to 6 carbon atoms). Preferred carbon atom numbers for these alkyl, aryl, aralkyl and heterocyclic-alkyl groups and their concrete examples are those described above for R¹, R² and R³.

Particularly preferred epoxysuccinamide derivatives of the formula (1) and their physiologically acceptable salts are the following two cases.
1) an epoxysuccinamide derivative or its physiologically acceptable salt in which R² is isobutyl or isopropyl, R³ is a hydrogen atom, Y¹ is OR⁵ (R⁵ has the meaning defined above), and Y² is a hydrogen atom. Preferred carbon atom numbers for the alkyl, aryl, aralkyl and heterocyclic-alkyl groups in the groups of R⁵ and their concrete examples are those described above for R¹, R², and R³.
2) an epoxysuccinamide derivative or its physiologically acceptable salt in which R² is isobutyl or isopropyl, R³ is an aryl group having 6 to 20 carbon atoms, Y¹ is OR⁵ (R⁵ has the meaning defined above), and Y² is a hydrogen atom. Preferred carbon atom numbers for the alkyl, aryl, aralkyl and heterocyclic-alkyl groups in the groups of R⁵ and their concrete examples are those stated above for R¹ R², and R³_{.}

In the groups of Y¹ and Y² of the formula (1), each of the alkyl groups for R⁵ to R¹⁰ an have one or more substituents selected from the group consisting of hydroxyl, amino, alkylamino having 1-6 carbon atoms (e.g., methylamino, ethylamino, n-propylamino, and isobutylamino), dialkylamino having 2-12 carbon atoms in total (e.g., di-methylamino, methylethylamino and diethylamino), alkoxy having 1-6 carbon atoms (e.g., methoxy, ethoxy, n-propoxy, isopropoxy, and n-butoxy), carboxyl, alkoxycarbonyl having 2-7 carbon atoms (e.g., ethoxycarbonyl), carbamoyl, alkylaminocarbonyl having 2-7 carbon atoms (e.g, methylaminocarbonyl and ethylaminocarbonyl), dialkylaminocarbonyl having 3-13 carbon atoms in total (e.g., dimethylaminocarbonyl, diethylaminocarbonyl, methylethylaminocarbonyl and piperadinocarbonyl), and guanidino.

Each of the aryl groups and the heterocyclic groups for R¹ to R¹⁰ way have one or more substituents selected from the group consisting of alkyl having 1-6 carbon atoms (e.g., methyl, ethyl, n-propyl, isopropyl, and n-butyl), hydroxyl, amino, alkylamino having 1-6 carbon atoms (e.g., methylamino, ethylamino, and n-propylamino), dialkylamino having 2-12 carbon atoms in total (e.g., di-methylamino, methylethylamino and diethylamino), alkoxy having 1-6 carbon atoms (e.g., methoxy, ethoxy, n-propoxy and isopropoxy), halogen atom (e.g., fluoride atom, chloride atom, and bromide atom), haloalkyl (e.g., trifluoromethyl), cyano, nitro, carboxyl, alkoxycarbonyl having 2-7 carbon atoms (e.g., ethoxycarbonyl), carbamoyl, alkylaminocarbonyl having 2-7 carbon atoms (e.g., methylaminocarbonyl and ethylaminocarbonyl), dialkylaminocarbonyl having 3-13 carbon atoms in total (e.g., dimethylaminocarbonyl and methylethylaminocarbonyl), amidino, and guanidino.

The two carbon atoms of the oxirane ring of the aforementioned formula (1) both are asymmetric carbon atoms. The formula (1) means that the two carbonyl groups attached to the oxirane ring are in the trans conformation. Therefore, the epoxysuccinamide derivative of the invention can be either an optical isomer in the form of (T1) or (T2) mentioned below, or a mixture thereof.

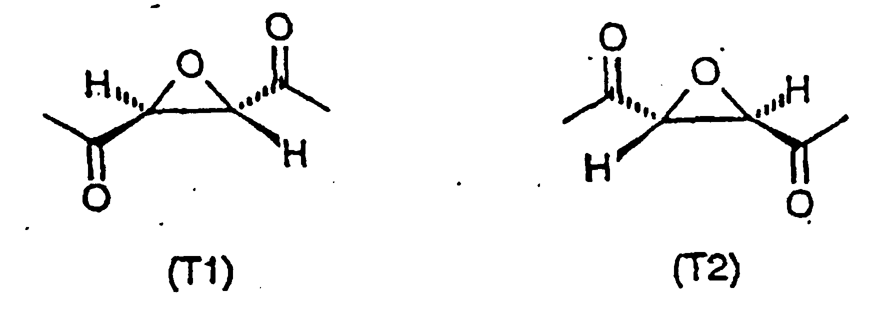

Examples of the epoxysuccinamide derivatives of the invention are set forth in the following Table 1 (Tables 1-1, 1-2, 1-3, and 1-4), wherein R¹, R², R³, X, Y¹ and Y² mean those shown in the aforementioned formula (1). In the following Table 1, each symbol means the following: H: hydrogen, Me: methyl, Et: ethyl, Ph: phenyl, Bn: benzyl, iPr: isopropyl, iBu: isobutyl, sBu: sec-butyl, tBu: tert-butyl, cHex: cyclohexyl, 4-MePh: 4-methylphenyl, 4-ClPh: 4-chlorophenyl, 4-tBuPh: 4-tert-butylphenyl, 4'-HOBn: 4'-hydroxybenzyl.

**Table 1-1**

| No. | R¹ | R² | R³ | X | Y¹ | Y² |
|---|---|---|---|---|---|---|
| 1 | Et | iBu | Ph | O | OH | H |
| 2 | H | iBu | Ph | O | OH | H |
| 3 | iPr | iBu | Ph | O | OH | H |
| 4 | Et | iBu | Ph | NH | OH | H |
| 5 | Ph | iBu | Ph | NMe | OH | H |
| 6 | Et | iBu | 4-MePh | O | OH | H |
| 7 | Et | iBu | 4-ClPh | O | OH | H |
| 8 | Et | iBu | iPr | O | OH | H |
| 9 | Et | sBu | Ph | O | OH | H |
| 10 | Et | Ph | Ph | O | OH | H |
| 11 | Et | iBu | Ph | O | OMe | H |
| 12 | Et | iBu | Ph | O | OEt | H |
| 13 | Et | iBu | H | O | OH | H |
| 14 | Et | iBu | Me | O | OH | H |
| 15 | Et | iBu | Ph | O | =O (together with Y²) | |
| 16 | Et | iPr | Ph | O | =O (together with Y²) | |
| 17 | Et | iPr | Ph | O | =S (together with Y²) | |
| 18 | H | sBu | Ph | O | OH | H |
| 19 | H | Ph | Ph | O | OH | H |
| 20 | H | iBu | Ph | O | OMe | H |
| 21 | H | iBu | H | 0 | OH | H |

**Table 1-2**

| No. | R¹ | R² | R³ | X | Y¹ | Y² |
|---|---|---|---|---|---|---|
| 22 | Et | iBu | Ph | O | acetoxy | H |
| 23 | Et | iBu | Ph | O | benzoyloxy | H |
| 24 | iPr | iBu | Ph | O | OMe | H |
| 25 | Bn | iBu | Ph | O | OMe | H |
| 26 | Et | iBu | Ph | O | benzyloxy | H |
| 27 | Et | iBu | H | O | benzyloxy | H |
| 28 | Et | iBu | H | O | 4'-chlorobenzyloxy | H |
| 29 | Et | iBu | H | O | 2'-methylbenzyloxy | H |
| 30 | Et | iBu | H | O | 3',4',5'-trimethoxybenzyloxy | H |
| 31 | Et | iPr | H | O | 2'-chlorobenzyloxy | H |
| 32 | Et | iPr | H | O | 4'-methylbenzyloxy | H |
| 33 | Et | iBu | H | O | 4'-amidinobenzyloxy | H |
| 34 | Et | iBu | H | O | 4'-guadininobenzyloxy | H |
| 35 | Et | iBu | H | O | carboxymethoxy | H |
| 36 | Et | iBu | H | O | (2-ethoxycarbonylethyl)oxy | H |
| 37 | Et | iBu | H | O | (1-piperadinylcarbonyl)methoxy | H |
| 38 | Et | iBu | H | O | isobutylamino | H |

**Table 1-3**

| No. | R¹ | R² | R³ | X | Y¹ | Y² |
|---|---|---|---|---|---|---|
| 39 | Et | iBu | H | O | diethylamino | H |
| 40 | Et | iBu | H | O | benzylamino | H |
| 41 | Et | iBu | H | O | benzoylamino | H |
| 42 | Et | iBu | H | O | benzylcarbonylamino | H |
| 43 | Et | iBu | H | O | isobutoxy | H |
| 44 | Et | Bn | H | O | benzyloxy | H |
| 45 | Et | Bn | H | O | isobutoxy | H |
| 46 | Et | 4'-HOBn | H | O | benzyloxy | H |
| 47 | Et | iBu | H | O | diphenylmethoxy | H |
| 48 | H | iBn | Ph | O | benzyloxy | H |
| 49 | H | Bn | H | O | benzyloxy | H |
| 50 | H | iBu | H | O | benzyloxy | H |
| 51 | H | iBu | H | O | (4-guanidinobutyl)oxy | H |
| 52 | H | iBu | H | O | (2-ethylaminoethyl)oxy | H |
| 53 | H | iBu | H | O | (2-diethylaminoethyl)oxy | H |

**Table 1-4**

| No. | R¹ | R² | R³ | X | Y¹ | Y² |
|---|---|---|---|---|---|---|
| 54 | Et | iBu | cHex | O | OH | H |
| 55 | Et | iBu | cHex | O | OMe | H |
| 56 | Et | iBu | H | O | 2'-methylbenzyloxy | H |
| 57 | Et | iBu | H | O | 2', 6'-dimethylbenzyloxy | H |
| 58 | Et | iBu | H | O | 4'-isopropylbenzyloxy | H |
| 59 | Et | iBu | H | O | 2'- chlorobenzyloxy | H |
| 60 | Et | iBu | H | O | 4'-trifluoromethylbenzyloxy | H |
| 61 | Et | iBu | H | O | 4'-cyanobenzyloxy | H |
| 62 | Et | iBu | H | O | 3'-aminobenzyloxy | H |
| 63 | Et | iBu | H | O | (3-pyridyl)methoxy | H |
| 64 | Et | iBu | H | O | (3-thienyl)methoxy | H |
| 65 | Et | iBu | H | O | (2-benzoimidazolyl)methoxy | H |
| 66 | Et | iBu | H | O | (1-naphthyl)methoxy | H |
| 67 | Et | iBu | H | O | 2-naphthyloxy | H |
| 68 | Et | iBu | H | O | phenoxy | H |
| 69 | Et | iBu | H | O | 2-phenylethoxy | H |
| 70 | Et | iBu | H | O | 3-phenylpropoxy | H |
| 71 | Et | iBu | H | O | ethoxy | H |
| 72 | Et | iBu | H | O | (3-methylbutyl) oxy | H |
| 73 | Et | iBu | H | O | hexyloxy | H |
| 74 | Et | iBu | H | O | cyclopropylmethoxy | H |
| 75 | Et | iBu | H | O | cyclohexylmethoxy | H |
| 76 | Et | iBu | H | O | (2-methyl-2-propenyl) oxy | H |
| 77 | Et | iBu | H | O | (3-methyl-2-butenyl) oxy | H |
| 78 | Et | iBu | H | O | 2-methoxyethoxy | H |
| 79 | Et | iBu | H | O | (dimethylcarbamoyl)methoxy | H |
| 80 | Et | iBu | H | O | 3-(4-benzyl-1-piperadinyl)propoxy | H |
| 81 | Et | iBu | H | O | 4-diethylaminobutyloxy | H |
| 82 | iPr | iBu | H | O | isobutoxy | H |
| 83 | tBu | iBu | H | O | isobutoxy | H |
| 84 | cHex | iBu | H | O | isobutoxy | H |
| 85 | Ph | iBu | H | O | isobutoxy | H |
| 86 | 4-tBuPh | iBu | H | O | isobutoxy | H |
| 87 | H | iBu | H | O | isobutoxy | H |
| 88 | Et | iPr | H | O | isobutoxy | H |
| 89 | Et | sBu | H | O | isobutoxy | H |
| 90 | Et | iPr | H | O | benzyloxy | H |
| 91 | Et | Bn | H | O | benzyloxy | H |
| 92 | Et | iBu | H | O | 2-methylpropionylamino | H |
| 93 | Et | iBu | H | O | hexanoylamino | H |
| 94 | Et | iBu | H | O | N-benzyl-N-methylamino | H |
| 95 | Et | iBu | H | O | N-hexyl-N-methylamino | H |

The epoxysuccinamide derivative can be employed in the form of a physiologically acceptable salt. For example, in the case that R¹ is a hydrogen atom and X is -O-, it forms a salt with an alkali metal (e.g., sodium or potassium), an alkaline earth metal (e.g., calcium), or an organic amine (e.g., lysine or meglumine).

Processes for preparing epoxysuccinamide derivatives of the invention are described below. The epoxysuccinamide derivative of the invention can be prepared from the known compound by a process involving production of amide-bonding, esterification, or hydrolysis. The respective reaction schemes are illustrated below.

### 1) Production of amide-bonding

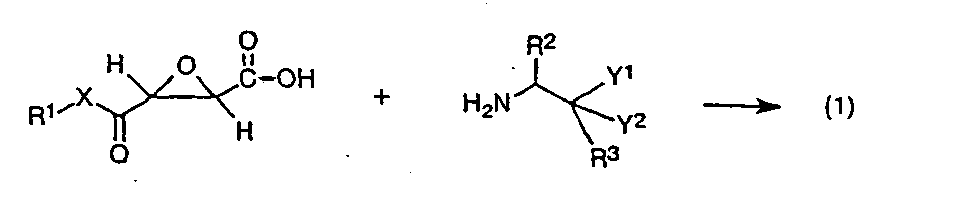

### 2) Production of amide-bonding (case of X = -NR⁴-)

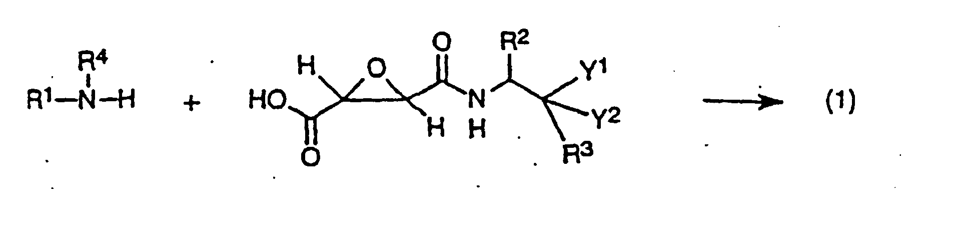

### 3) Esterification (case of R⁵ is alkylcarbonyl, arylcarbonyl, aralkylcarbonyl, heterocycliccarbonyl, or heterocyclic-alkylcarbonyl)

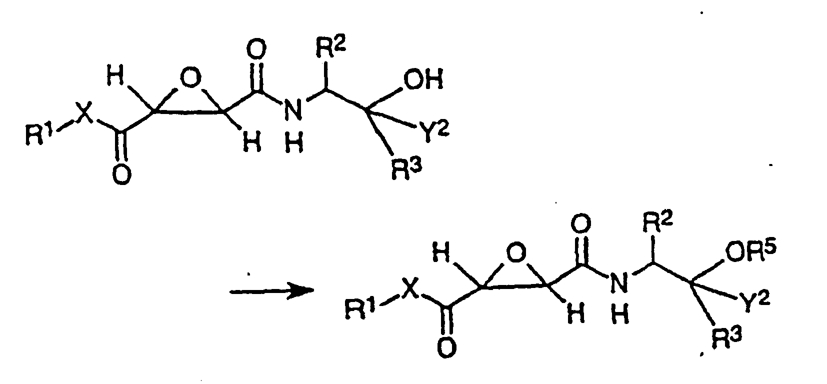

### 4) Esterification (case of X = -O- and R¹ is a group other than hydrogen)

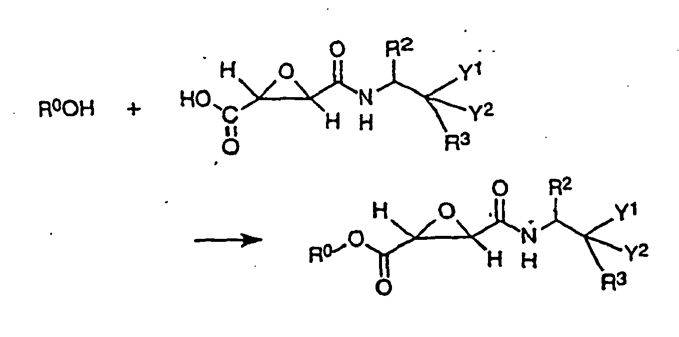

(in the above-illustrated reaction scheme, R⁰ is the same as R¹ except that hydrogen is not included)

### 5) Hydrolysis (case of X = -O- and R¹ = hydrogen)

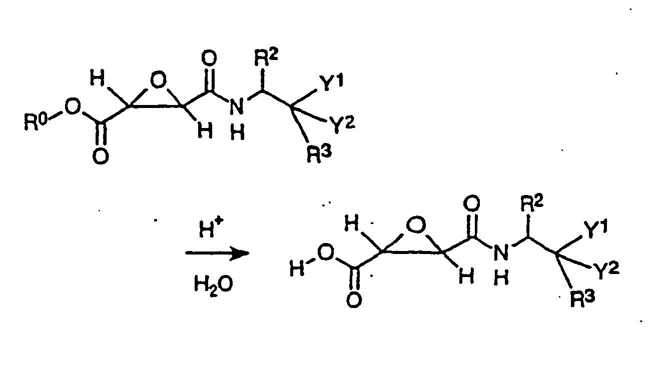

(in the above-illustrated reaction scheme, R⁰ is the same as R¹ except that hydrogen is not included)

In performing the above-illustrated reactions, groups such as R¹ and/or Y¹ can be protected by a known protective group, if necessary.

In the preparation of an epoxysuccinamide derivative of the invention, processes for preparing epoxysuccinamide derivatives described in Japanese Patent Publication No. 61-55509, Japanese Patent Provisional Publication No. 52-31024, H8-41043, H8-104684 and WO 96/30354 can be utilized. As for the detailed reaction conditions for preparing epoxysuccinamide derivatives of the invention, representative reaction conditions are stated in a great number of the working examples.

The epoxysuccinamide derivative of the invention can be administered by either oral or parenteral route. The oral formulation can be prepared in the form of tablets, capsules, powder, granules, or syrup. The parenteral administration can be performed through mucosal membrane, body surface, blood vessel, or tissue. The mucosal administration can be performed using ophthalmic solutions, inhalant, spray, or suppository. The surface administration can be performed using ointment. The administration through blood vessel and tissue can be performed using injections.

The oral formulation can be prepared using conventional excipient, disintegrator, binder, lubricant, dye and diluent. The excipient generally is glucose or lactose. The disintegrator can be starch or carboxymethyl cellulose calcium. The lubricant can be magnesium stearate or talc. The binder can be hydroxypropyl cellulose, gelatin, or polyvinyl alcohol.

The pharmaceutical composition for parenteral administration can be prepared in the conventional way. For instance, the injection can be prepared using ordinary distilled water for injection, saline, or Ringer's solution.

The dosage of the epoxysuccinamide derivative of the invention is in the range of 0.01 mg to 100 mg/day for adult in the case of using injection. In the case of oral administration, the dosage is in the range of 0.1 to 1 g/day for adult. The dosage can be increased or decreased depending on age, race, and conditions of patients.

### Examples

### [Experimentally induced cerebral aneurysm model]

Cerebral aneurysms were induced as previously described by Nagata et al.(Nagata I, Handa H, Hashimoto N, Hazama F. Experimentally induced cerebral aneurysms in rats: Part VI. Hypertension. Surg Neurol. 1980;14:477-479). After the induction of pentobarbital anesthesia (50mg/kg), the left common carotid artery and posterior branches of the bilateral renal arteries were ligated at the same time with 10-0 nylon in 7 week-old male SD (Sprague-Dawley) rats. Animals were fed special food containing 8% sodium chloride and 0.12% β-aminopropionitrile (an inhibitor of lysyl oxidase that catalyzes the cross-linking of collagen and elastin). Blood pressure was measured by tail-cuff method. Animal care and experiments complied with Japanese community standards on the care and use of laboratory animals.

### [Immunohistochemistry]

Three months after aneurysm induction, all rats (n=10) were deeply anesthetized and perfused transcardinally with 4% paraformaldehyde. As a control, age-matched male Sprague-Dawley rats were euthanized as described above. The anterior cerebral artery / olfactory artery (ACA/OA) bifurcation was stripped, embedded and cut into 5µm sections. After blocking with 5% Donkey serum, primary antibodies were incubated for one hour at room temperature followed by incubation with fluorescence labeled secondary antibodies for one hour at room temperature. The secondary antibodies were FITC-conjugated donkey anti-goat IgG antibody, FITC-conjugated donkey anti-rabbit IgG antibody, Cy3-conjugated donkey antimouse IgG antibody, and Cy3-conjugated donkey anti-rabbit IgG antibody. Then the slides were covered with PERMAFLUOR (Immunotec) and excited for fluorescence by illumination through a fluorescence microscope system (Olympus). The primary antibodies used in the present study are goat polyclonal anti-cathepsin B antibody, goat polyclonal anti-cathepsin K antibody, goat polyclonal anti-cathepsin S antibody, rabbit polyclonal anti-cystatin C antibody, mouse monoclonal anti-CD68 antibody, mouse monoclonal anti-smooth muscle α-actin antibody, and rabbit anti-endothelial nitric oxide synthase (eNOS) polyclonal antibody.

### [RT-PCR]

One (n=6) or three (n=6) months after aneurysm induction, rats were euthanized described above. Total RNA from the whole Willis ring was isolated using RNeasy Fibrous Tissue Mini Kit (QIAGEN). Extraction was performed according to the manufacture's directions. Total RNA was converted into cDNA using Sensiscript reverse transcriptase (QIAGEN). The conditions for the cDNA synthesis were: one hour at 37°C followed by heating at 93°C for five minutes. PCR was performed using HotStar Taq polymerase (QIAGEN) and iCycler (BIO-RAD). β-Actin was used as an internal control. The primer sets used were shown below.
- Cathepsin B:: forward 5'-aaatcaggcgtatacaagcatga-3'
reverse 5'-gcccagaatgcggatgg-3'
- Cathepsin K:: forward 5'-cccagactccatcgactatcg-3'
reverse 5'-ctgtaccctctgcacttagctgcc-3'
- Cathepsin S:: forward 5'-acgagcatcgactcagaagc-3'
reverse 5'-tagccaaccacgagaacacc-3'
- Cyctatin C:: forward 5'-ggattctcgactcagagtatcc-3'
reverse 5'-gactgcacgtcttggacggacg-3'
- β-Actin:: forward 5'-aagcaatgctgtcaccttccc-3'
reverse 5'-aagtccctcaccctcccaaaag-3'

The condition for PCR reactions was 45cycles of 95°C thirty seconds for the denaturation, 53°C thirty seconds for the annealing, 72°C thirty seconds for the extension. PCR products were separated by the electrophoresis in 2% agarose gel. Densitometric analysis includes data of 6 samples per group.

### [Cathepsin protease inhibitor]

As a cathepsin protease inhibitor, monosodium (2S,3S)-3-[[(1S)-1-isobutoxymethyl-3-methylbutyl]carbamoyl]-oxirane-2-carboxylate shown below was used.

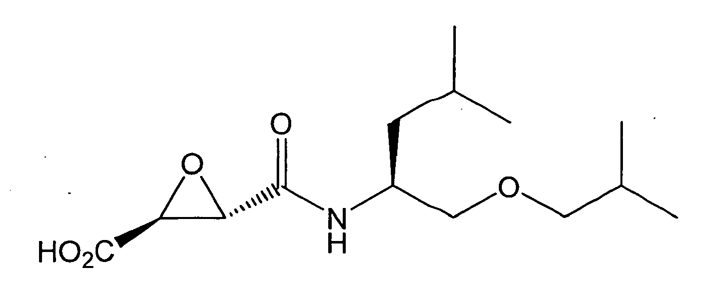

The inhibitor strongly inhibits cathepsin K, and also inhibits cathepsin B, or S. IC 50 for cathepsin K is 34.5 nmol/L, for B is 284 nmol/L, and for S is 186 nmol/L. The inhibitor did not inhibit other proteases such as matrix metalloproteases.

A total of 18 rats were orally given 50mg/kg/day of the inhibitor. Immediately after aneurysm induction, rats were fed food with (n=18) or without (n=21) 50mg/kg/day of the inhibitor, and sacrificed after three months. The ACA/OA bifurcation was stripped and observed under a light microscope after Elastica van Gieson staining. Early aneurysmal change refers to a lesion with the discontinuity of the internal elastic lamina without apparent outward bulging of the arterial wall. Advanced aneurysm refers to an obvious outward bulging of the arterial wall with the fragmentation or disappearance of the internal elastic lamina. After immunohistochemistry for CD68, the number of CD68-positive cells was counted on each section. Three independent researchers assessed the histopathological changes.

### [Measurement of enzyme activity]

In order to examine cathepsin B activity in aneurysmal walls, azo-coupling method (in situ) was performed. Three months after aneurysm induction, rats (n=6 in each group) were euthanized as described above. Five µm frozen sections were incubated for one hour at 37°C with 3.2 mM Z-arg-arg-4-Methoxy-2-Naphthylamine (Cosmo Bio) in 100mM potassium phosphate buffer containing 10% polyvinyl alcohol, 10 mM dithiothreitol, 2.7 mM L-Cysteine and 1.3 mM EDTA (Sigma). After then, the slides were placed in 125 mg/ml of N-ethylmaleimide in potassium phosphate buffer following at 55°C for 5 minutes, followed by incubation with Fast Blue Solution (Fast Blue 21mg/100ml of potassium phosphate solution, Sigma) for thirty minutes and 0.1M cupric sulfate solution for 15 minutes at room temperature. After washing with physiological saline, the slides were covered with PERMAFLUOR (Immunotec).

### [Measurement of collagenase activity]

Total protein from the whole Willis ring of the control group and the group treated with cysteine protease inhibitor was purified by Bio-Plex Cell Lysis Kit (Bio-Rad) according to the manufacture's directions. One hundred fifty µg of protein was used in one experiment (n=5 in each group). Collagenase activity was measured using Collagenase Activity Measurement Kit (Life Laboratory). Briefly, fluorescence labeled type I or IV collagen was incubated with the lysate of the whole Willis ring for one hour at 37°C. Undigested fluorescence labeled collagen was extracted in ethanol solution and measured by fluorospectrometer with excitation 496 nm and measurement 520 nm. Collgenase activity (U/ml) was calculated by the fluorescence intensity according to the formula given in the manufacture's direction.

### [Immunohistochemistry for human samples]

Human cerebral aneurysm samples were obtained from 4 patients who underwent neck clipping for unruptured aneurysms with informed consent. As a control, the middle cerebral artery (MCA) (n=2) obtained at the STA-MCA bypass surgery was used. Four µm paraffin sections were cut and mounted on slides. After deparaffinization and blocking of endogenous peroxidase activity with 0.3% H₂O₂, primary antibodies for cathepsins and cystatin C (the same as used in the rat study) were incubated for 12 hours at 4°C followed by incubation with biotin-labeled secondary antibodies for 30 minutes at room temperature. After then, the slides were incubated with streptavidin-conjugated peroxidase. Finally the signal was detected by 3,3'-diaminobenzidine system. Nuclear staining was performed by hematoxylin solution. As a negative control, immunohistochemistry without primary antibodies was perfomed. For double staining, the slides were incubated with primary antibodies for smooth muscle α-actin, CD68 or von Willebrand factor for 30 minutes at room temperature, followed by the incubation with alkaline phosphatase-labeled secondary antibodies and Fast red solution.

### [Statistical Analysis]

The values were expressed as means ± Standard deviation (SD). Statistical analysis was performed by using Student's t-test for a two-group comparison and one-way ANOVA followed by Fisher test for a multiple comparison. The incidence of aneurysmal changes was analyzed by the use of Fisher's exact test. Differences were considered statistically significant at P<0.05.

### [Expression of cathepsins and cystatin C in experimentally induced cerebral aneurysms in rats]

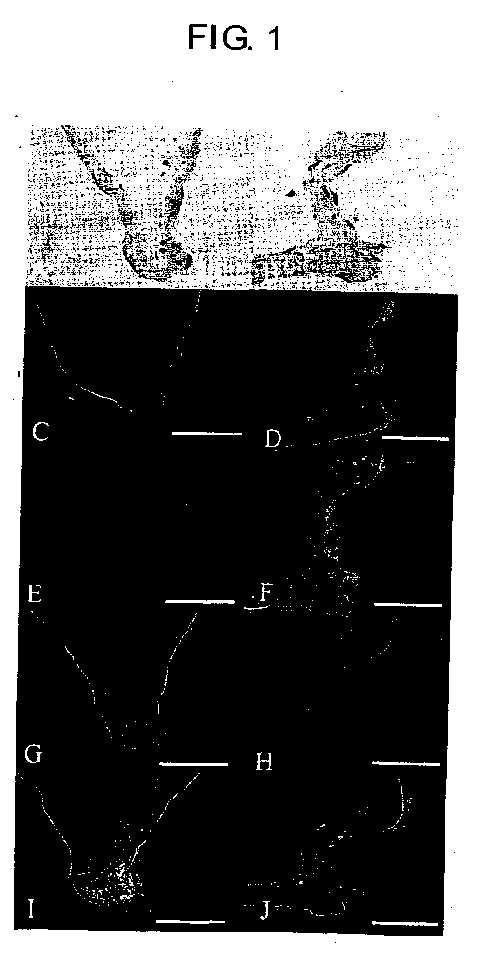

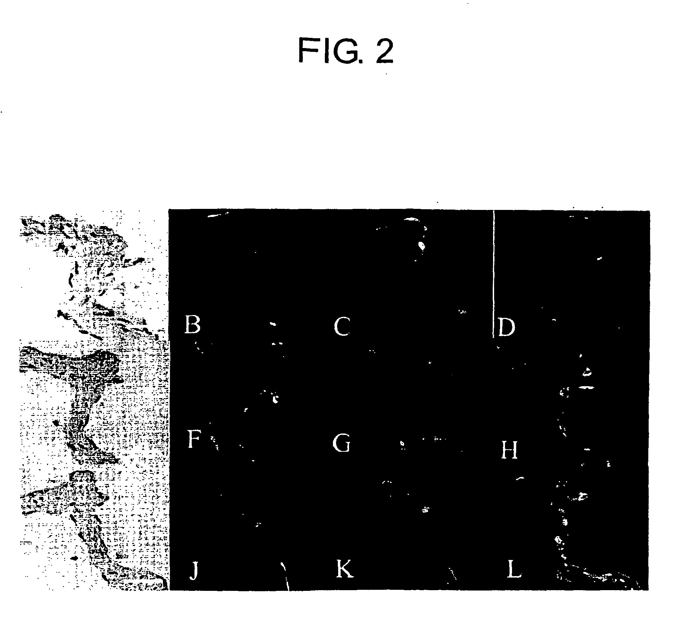

Three months after aneurysm induction, (19/21) of rats developed an advanced aneurysm at the ACA/OA bifurcation (data not shown). Cathepsins B, K and S (Figure 1 D, F, H) were highly expressed in the intima and media of aneurysmal walls. Small amounts of Cathepsin B and S expression could be seen in the adventitia (Figure 1 D, H). Cathepsin expression could not be detected in arterial walls of control rats (Figure 1 C, E, G). In contrast, cystatin C was abundantly expressed mainly in the media of arterial walls in control rats (Figure 1 I), and scarcely expressed in aneurysmal walls (Figure 1 J). All three kinds of cells (endothelium, smooth muscle cell, and macrophage) expressed cathepsin B, K and S in aneurysmal walls (Figure 2). The main source of cathepsin B was macrophages (Figure 2 D) and that of cathepsin K and S was smooth muscle cells (Figure 2 F-L).

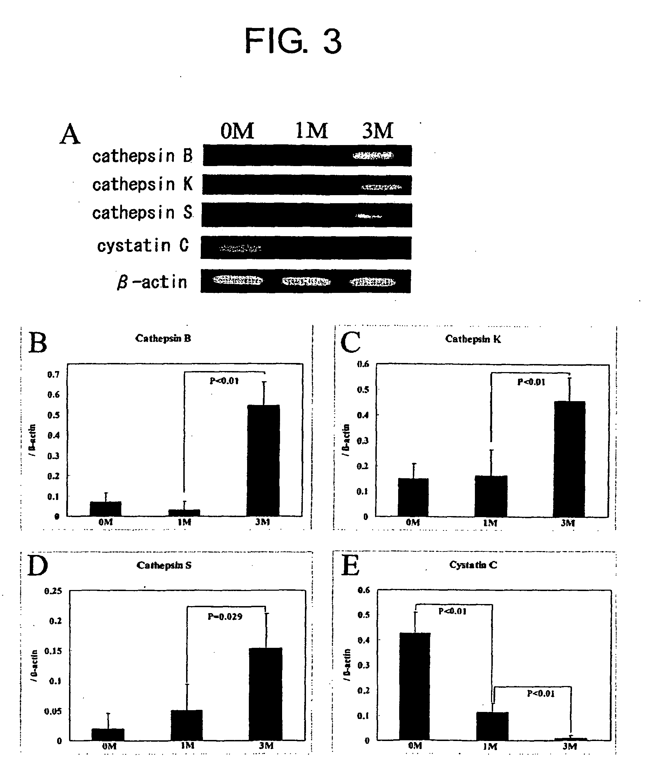

### [Expression of mRNA in experimentally induced cerebral aneurysms in rats (Figure 3)]

In the control arterial wall, only small amounts of cathepsin B, K and S mRNA were expressed. Their expression did not increase one month after aneurysm induction and was markedly upregulated after three months. In contrast, cystatin C mRNA was highly expressed in the control arterial walls and its expression decreased with aneurysm progression.

### [The effect of a cysteine protease inhibitor on aneurysm formation and progression]

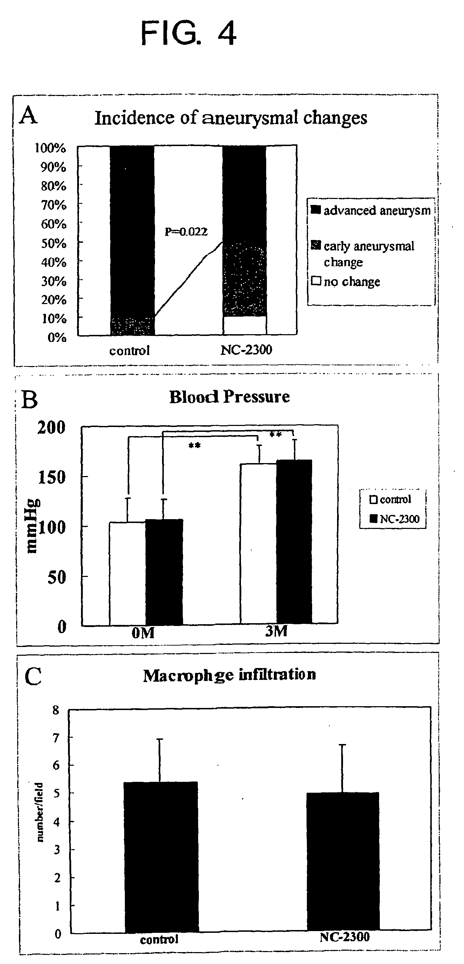

In the next, it was examined whether the cysteine protease inhibitor could prevent the initiation and progression of cerebral aneurysms in our model. The serum concentration of the cysteine protease inhibitor reached the value sufficient for inhibition of cysteine cathepsins (628.5±22.3 nmol/L). In the control group, 19 of 21 rats, developed advanced aneurysms and 2 showed early aneurysmal changes. In the cysteine protease inhibitor treated rats (50mg/kg/day), only 5 of 10 rats developed advanced aneurysms and 4 showed early aneurysmal changes. The incidence of all aneurysmal changes was not different between the two groups. However, the rate of advanced aneurysms was significantly lower in the cysteine protease inhibitor group than in the control group (P=0.022) (Figure 4 A). In both groups, systemic blood pressure was elevated after three months of aneurysm induction, but there was no significant difference between the control group (160.7±21.1 mmHg, n=20) and the cysteine protease inhibitor group (164.6±20.7 mmHg, n=10) (Figure 4 B). Macrophage infiltration per 50 µm x 50 µm field around aneurysms did not differ between the control group (5.3±1.5 cells/field, n=14) and the cysteine protease inhibitor group (4.9±1.7 cells/field, n=10) (Figure 4 C)

### [Cathepsin B activity in experimentally induced cerebral aneurysms in rats with inhibitor treatment]

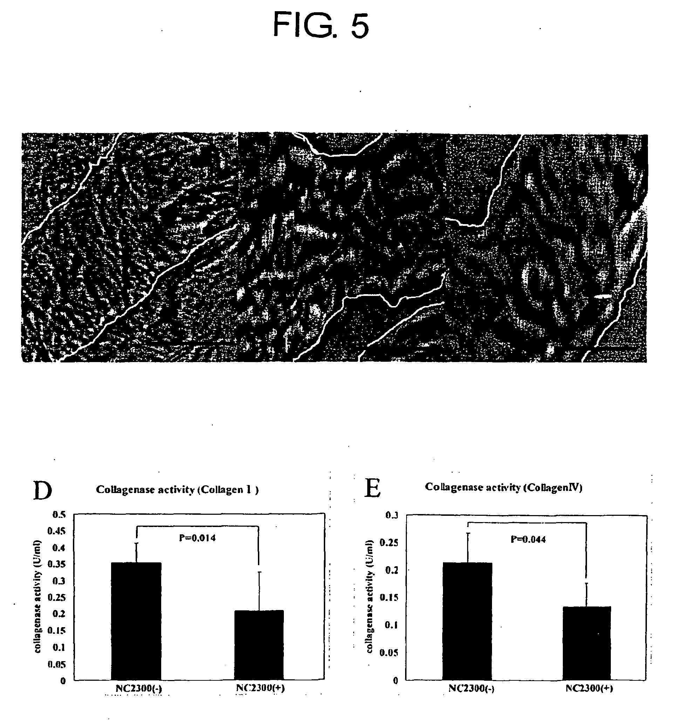

Three months after aneurysm induction, cathepsin B activity was prominent at the aneurysmal walls (Figure 5 B). Its activity was reduced in the cysteine protease inhibitor-treated rats (Figure 5 C). In the control arterial wall, cathepsin B activity could not be detected (Figure 5 A).

### [Collagenase activities in experimentally induced cerebral aneurysms in rats with inhibitor treatment]

In the cysteine protease inhibitor-treated rats (n=5), both collagenase I and IV activities were significantly lower than in the control group (N=5) (collagenase I: P=0.014, collagenase IV: P=0.044) (Figure 5 D, E).

### [Expression of cathepsins and cystatin C in human cerebral aneurysms]

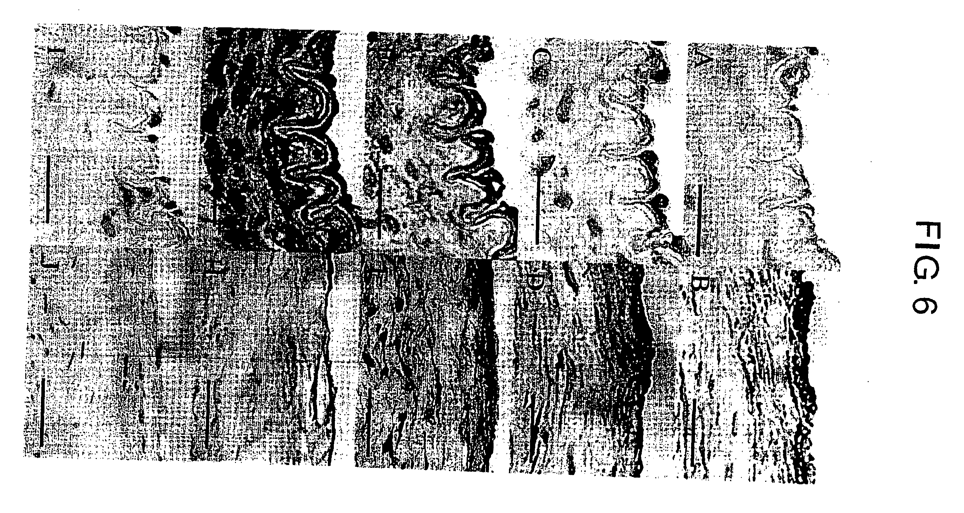

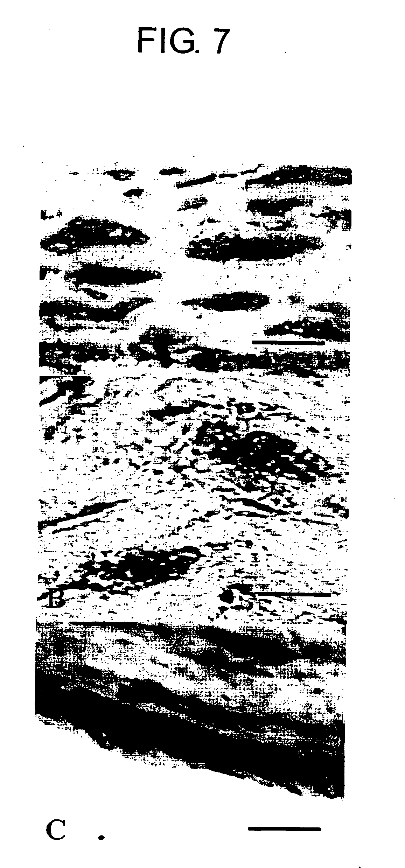

Cathepsin B, K and S were highly expressed in endothelial cell layer and the media in aneurysmal walls (Figure 6 B, D, F). In the control artery, cathepsins were only faintly expressed in the endothelial cell layer (Figure 6 A, C, E). Cystatin C was highly expressed in the intima and media of cerebral arterial walls (Figure 6 G) and its expression was reduced in aneurysmal walls (Figure 6 H). Negative controls without the incubation with primary antibodies showed no positive signal in both of the control and the aneurysm (Figure 6 I, J). Double immunohistostaining demonstrated cathepsin S expression in all of smooth muscle cells, macrophages, and endothelial cells (Figure 7). Cathepsin B and K were also expressed in all three kinds of cells.

### Industrially applicable field

Extensive degradation of elastin and collagen is found in the arterial wall of cerebral aneurysms. Revealing the molecular mechanisms that trigger and promote the degradation of extracellular matrix provides new therapeutic modalities for cerebral aneurysm in the present invention.

Treatment with the cysteine protease inhibitor dramatically reduced the incidence of advanced cerebral aneurysms, not affecting systemic blood pressure and macrophage infiltration. The results suggest that cysteine cathepsins promotes progression of cerebral aneurysm.

Cysteine cathepsins can be a treatment target for preventing aneurysm growth and rupture. Therefore, a cysteine protease inhibitor can be used as an effective medicament for treatment and prophylaxis of cerebral aneurysms.

### Brief description of the drawings

### [Figure 1]

Photographs showing cathepsins and cystatin C expression in experimentally induced cerebral aneurysmal wall and control arterial wall in rats

### [Figure 2]

Photographs showing expression of cathepsins in endothelium, smooth muscle cell, and macrophage of experimentally induced cerebral aneurysms in rats

### [Figure 3]

Figures showing expression of mRNA in experimentally induced cerebral aneurysms in rats

### [Figure 4]

Figures showing effect of a cysteine protease inhibitor on initiation and progression of cerebral aneurysms

### [Figure 5]

Figures showing cathepsin B and collagenase activity in aneurysmal walls

### [Figure 6]

Photographs showing cathepsins and cystatin C expression in human cerebral aneurysmal wall and control arterial wall

### [Figure 7]

Photographs showing expression of cathepsin S in endothelium, smooth muscle cell, and macrophage of human cerebral aneurysmsal wall

### Figure legends

Figure 1 A
   Control arterial wall
Figure 1 B
   Aneurysmal wall
Figure 1 C
   Cathepsin B expression in control arterial wall
Figure 1 D
   Cathepsin B expression in aneurysmal wall
Figure 1 E
   Cathepsin K expression in control arterial wall
Figure 1 F
   Cathepsin K expression in aneurysmal wall
Figure 1 G
   Cathepsin S expression in control arterial wall
Figure 1 H
   Cathepsin S expression in aneurysmal wall
Figure 1 I
   Cystatin C expression in control arterial wall
Figure 1 J
   Cystatin C expression in aneurysmal wall Bar length in Figure 1
   50 µm
Figure 2 A, E, I
   Aneurysmal wall
Figure 2 B
   Cathepsin B expression in endothelium
Figure 2 C
   Cathepsin B expression in macrophage
Figure 2 D
   Cathepsin B expression in smooth muscle cell
Figure 2 F
   Cathepsin K expression in endothelium
Figure 2 G
   Cathepsin K expression in macrophage
Figure 2 H
   Cathepsin K expression in smooth muscle cell
Figure 2 J
   Cathepsin S expression in endothelium
Figure 2 K
   Cathepsin S expression in macrophage
Figure 2 L
   Cathepsin S expression in smooth muscle cell Bar length in Figure 2
   50 µm
Figure 3 A
   Results of electrophoresis for PCR products
Figure 3 B
   Expression of cathepsin B mRNA
Figure 3 C
   Expression of cathepsin K mRNA
Figure 3 D
   Expression of cathepsin S mRNA
Figure 3 E
   Expression of cystatin C mRNA
Figure 4 A
   Incidence of aneurismal changes
Figure 4 B
   Blood pressure
Figure 4 C
   Macrophage infiltration
Figure 5 A
   Control cathepsin B activity
Figure 5 B
   Cathepsin B activity after aneurysm induction without treatment of cysteine protease inhibitor
Figure 5 C
   Cathepsin B activity after aneurysm induction with treatment of cysteine protease inhibitor
Figure 5 D
   Collagenase I activity
Figure 5 E
   Collagenase IV activity
   Bar length in Figure 5
   50 µm
Figure 6 A
   Cathepsin B expression in control arterial wall
Figure 6 B
   Cathepsin B expression in aneurysmal wall
Figure 6 C
   Cathepsin K expression in control arterial wall
Figure 6 D
   Cathepsin K expression in aneurysmal wall
Figure 6 E
   Cathepsin S expression in control arterial wall
Figure 6 F
   Cathepsin S expression in aneurysmal wall
Figure 6 G
   Cystatin C expression in control arterial wall
Figure 6 H
   Cystatin C expression in aneurysmal wall
Figure 6 G
   Cystatin C expression in control arterial wall
Figure 6 H
   Cystatin C expression in aneurysmal wall
Figure 6 I
   Control arterial wall (negative control)
Figure 6 J
   Aneurysmal wall (negative control)
   Bar length in Figure 6
   50 µm
Figure 7 A
   Cathepsin S expression in endothelium
Figure 7 B
   Cathepsin S expression in macrophage
Figure 7 C
   Cathepsin S expression in smooth muscle cell

## Claims

1. A medicament for treatment or prophylaxis of cerebral aneurysms, wherein the medicament contains a cysteine protease inhibitor as an active ingredient.

2. The medicament as defined in claim 1, which is directed to treatment or prophylaxis of progression or rupture of cerebral aneurysms.

3. The medicament for treatment or prophylaxis as defined in claim 1, wherein the cysteine protease inhibitor is an inhibitor of cathepsin B, cathepsin K, or cathepsin S.

4. The medicament for treatment or prophylaxis as defined in claim 1, wherein the cysteine protease inhibitor is N-[1-[(cyanomethyl)carbamoyl]cyclohexyl]-4-(4-propylpyperazin-1-yl)benzamide, N-[(1S)-3-methyl-1-[[(4S,7R)-7-methyl-3-oxo-1-(pyridin-2-ylsulfonyl)hexahydro-1H-azepin-4-yl]carbamoyl]butyl]-1-benzofuran-2-carboxamide, (2R)-N-cyanomethyl-4-methyl-2-(4'-piperazin-1-yl-1,1'-biphenyl-3-yl)pentanamide, N-[3-[(2Z)-2-(3-methyl-1,3-thiazolin-2-ylidene)hydrazino]-2,3-dioxo-1-tetrahydro-2H-pyran-4-ylpropyl]cycloheptanecarboxamide, N-cyanomethyl-4-methyl-2-[2,2,2-trifluoro-1-(4'-methylsulfonyl-1,1'-biphenyl-4-yl)ethylamino]pentanamide, or monosodium (2S,3S)-3-[[(1S)-1-isobutoxymethyl-3-methylbutyl]carbamoyl]oxirane-2-carboxylate.

5. A medicament for treatment or prophylaxis of cerebral aneurysms, wherein the medicament contains an epoxysuccinamide derivative having the formula (1) or its physiologically acceptable salt as an active ingredient: 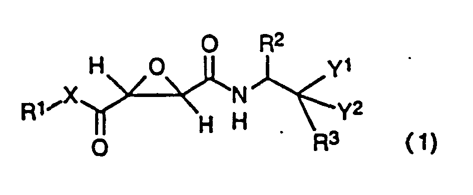 wherein R¹ represents a hydrogen atom, an alkyl group having 1 to 10 carbon atoms, an alkenyl group having 2 to 10 carbon atoms, an alkynyl group having 2 to 10 carbon atoms, an aryl group having 6 to 20 carbon atoms, an aralkyl group comprising an aryl group having 6 to 20 carbon atoms and an alkyl group having 1 to 6 carbon atoms, a heterocyclic group having 3 to 12 carbon atoms, or a heterocyclic-alkyl group comprising a heterocyclic group having 3 to 12 carbon atoms and an alkyl group having 1 to 6 carbon atoms; R² represents an alkyl group having 1 to 10 carbon atoms, an alkenyl group having 2 to 10 carbon atoms, an alkynyl group having 2 to 10 carbon atoms, an aryl group having 6 to 20 carbon atoms, an aralkyl group comprising an aryl group having 6 to 20 carbon atoms and an alkyl group having 1 to 6 carbon atoms, a heterocyclic group having 3 to 12 carbon atoms, or a heterocyclic-alkyl group comprising a heterocyclic group having 3 to 12 carbon atoms and an alkyl group having 1 to 6 carbon atoms; R³ represents a hydrogen atom, an alkyl group having 1 to 10 carbon atoms, an alkenyl group having 2 to 10 carbon atoms, an alkynyl group having 2 to 10 carbon atoms, an aryl group having 6 to 20 carbon atoms, an aralkyl group comprising an aryl group having 6 to 20 carbon atoms and an alkyl group having 1 to 6 carbon atoms, a heterocyclic group having 3 to 12 carbon atoms, or a heterocyclic-alkyl group comprising a heterocyclic group having 3 to 12 carbon atoms and an alkyl group having 1 to 6 carbon atoms; X represents -O- or -NR⁴- in which R⁴ is a hydrogen atom, an alkyl group having 1 to 10 carbon atoms, an aryl group having 6 to 20 carbon atoms, an aralkyl group comprising an aryl group having 6 to 20 carbon atoms and an alkyl group having 1 to 6 carbon atoms, a heterocyclic group having 3 to 12 carbon atoms, or a heterocyclic-alkyl group comprising a heterocyclic group having 3 to 12 carbon atoms and an alkyl group having 1 to 6 carbon atoms; Y¹ represents OR⁵ in which R⁵ is a hydrogen atom, an alkyl group having 1 to 10 carbon atoms, an aryl group having 6 to 20 carbon atoms, an aralkyl group comprising an aryl group having 6 to 20 carbon atoms and an alkyl group having 1 to 6 carbon atoms, an acyl group having 2 to 20 carbon atoms, a heterocyclic group having 3 to 12 carbon atoms, or a heterocyclic-alkyl group comprising a heterocyclic group having 3 to 12 carbon atoms and an alkyl group having 1 to 6 carbon atoms, SR⁶ in which R⁶ is a hydrogen atom, an alkyl group having 1 to 10 carbon atoms, an aryl group having 6 to 20 carbon atoms, an aralkyl group comprising an aryl group having 6 to 20 carbon atoms and an alkyl group having 1 to 6 carbon atoms, an acyl group having 2 to 20 carbon atoms, a heterocyclic group having 3 to 12 carbon atoms, or a heterocyclic-alkyl group comprising a heterocyclic group having 3 to 12 carbon atoms and an alkyl group having 1 to 6 carbon atoms, or NR⁷R⁸ in which R⁷ is a hydrogen atom, an alkyl group having 1 to 10 carbon atoms, an aryl group having 6 to 20 carbon atoms, an aralkyl group comprising an aryl group having 6 to 20 carbon atoms and an alkyl group having 1 to 6 carbon atoms, an acyl group having 2 to 20 carbon atoms, a heterocyclic group having 3 to 12 carbon atoms, a heterocyclicalkyl group comprising a heterocyclic group having 3 to 12 carbon atoms and an alkyl group having 1 to 6 carbon atoms, and R⁸ is a hydrogen atom, an alkyl group having 1 to 10 carbon atoms, an aryl group having 6 to 20 carbon atoms, an aralkyl group comprising an aryl group having 6 to 20 carbon atoms and an alkyl group having 1 to 6 carbon atoms, a heterocyclic group having 3 to 12 carbon atoms, or a heterocyclic-alkyl group comprising a heterocyclic group having 3 to 12 carbon atoms and an alkyl group having 1 to 6 carbon atoms; and Y² represents a hydrogen atom or an alkyl group having 1 to 10 carbon atoms; or Y¹ and Y² in combination with each other can form =O, =S, =N-R⁹ in which R⁹ is a hydrogen atom, an alkyl group having 1 to 10 carbon atoms, an aryl group having 6 to 20 carbon atoms, an aralkyl group comprising an aryl group having 6 to 20 carbon atoms and an alkyl group having 1 to 6 carbon atoms, a heterocyclic group having 3 to 12 carbon atoms, or a heterocyclic-alkyl group comprising a heterocyclic group having 3 to 12 carbon atoms and an alkyl group having 1 to 6 carbon atoms, or =N-OR¹⁰ in which R¹⁰ is a hydrogen atom, an alkyl group having 1 to 10 carbon atoms, an aryl group having 6 to 20 carbon atoms, an aralkyl group comprising an aryl group having 6 to 20 carbon atoms and an alkyl group having 1 to 6 carbon atoms, a heterocyclic group having 3 to 12 carbon atoms, or a heterocyclic-alkyl group comprising a heterocyclic group having 3 to 12 carbon atoms and an alkyl group having 1 to 6 carbon atoms; provided that each of the alkyl groups for R⁵ to R¹⁰ can have one or more substituents selected from the group consisting of hydroxyl, amino, an alkylamino group having 1 to 6 carbon atoms, a dialkylamino group having 2 to 12 carbon atoms in total, an alkoxy group having 1 to 6 carbon atoms, carboxyl, an alkoxycarbonyl group having 2 to 7 carbon atoms, carbamoyl, an alkylaminocarbonyl group having 2 to 7 carbon atoms, a dialkylaminocarbonyl group having 3 to 13 carbon atoms in total, and guanidine, and each of the aryl groups and the heterocyclic groups for R¹ to R¹⁰ can have one or more substituents selected from the group consisting of an alkyl group having 1 to 6 carbon atoms, hydroxyl, amino, an alkylamino group having 1 to 6 carbon atoms, a dialkylamino group having 2 to 12 carbon atoms in total, an alkoxy group having 1 to 6 carbon atoms, a halogen atom, a haloalkyl group having 1 to 6 carbon atoms, cyano, nitro, carboxyl, an alkoxycarbonyl group having 2 to 7 carbon atoms, carbamoyl, an alkylaminocarbonyl group having 2 to 7 carbon atoms, a dialkylaminocarbonyl group having 3 to 13 carbon atoms in total, amidino, and guanidino.

6. The medicament for treatment or prophylaxis as defined in claim 5, wherein R¹ represents a hydrogen atom, an alkyl group having 1 to 10 carbon atoms, an alkenyl group having 2 to 10 carbon atoms, an alkynyl group having 2 to 10 carbon atoms, an aryl group having 6 to 20 carbon atoms, an aralkyl group comprising an aryl group having 6 to 20 carbon atoms and an alkyl group having 1 to 6 carbon atoms, a heterocyclic group having 3 to 12 carbon atoms, or a heterocyclic-alkyl group comprising a heterocyclic group having 3 to 12 carbon atoms and an alkyl group having 1 to 6 carbon atoms; R² represents an alkyl group having 1 to 10 carbon atoms, an alkenyl group having 2 to 10 carbon atoms, an alkynyl group having 2 to 10 carbon atoms, an aryl group having 6 to 20 carbon atoms, an aralkyl group comprising an aryl group having 6 to 20 carbon atoms and an alkyl group having 1 to 6 carbon atoms, a heterocyclic group having 3 to 12 carbon atoms, or a heterocyclic-alkyl group comprising a heterocyclic group having 3 to 12 carbon atoms and an alkyl group having 1 to 6 carbon atoms; R³ represents a hydrogen atom, an alkyl group having 1 to 10 carbon atoms, an alkenyl group having 2 to 10 carbon atoms, an alkynyl group having 2 to 10 carbon atoms, an aryl group having 6 to 20 carbon atoms, an aralkyl group comprising an aryl group having 6 to 20 carbon atoms and an alkyl group having 1 to 6 carbon atoms, a heterocyclic group having 3 to 12 carbon atoms, or a heterocyclic-alkyl group comprising a heterocyclic group having 3 to 12 carbon atoms and an alkyl group having 1 to 6 carbon atoms; X represents -O- or -NR⁴- in which R⁴ is a hydrogen atom, an alkyl group having 1 to 10 carbon atoms, an aryl group having 6 to 20 carbon atoms, an aralkyl group comprising an aryl group having 6 to 20 carbon atoms and an alkyl group having 1 to 6 carbon atoms, a heterocyclic group having 3 to 12 carbon atoms, or a heterocyclic-alkyl group comprising a heterocyclic group having 3 to 12 carbon atoms and an alkyl group having 1 to 6 carbon atoms; Y¹ represents hydroxyl, an alkoxy group having 1 to 6 carbon atoms, acetoxy, or an aralkyloxy group comprising an aryl group having 6 to 20 carbon atoms and an alkyl group having 1 to 6 carbon atoms; and Y² represents a hydrogen atom or an alkyl group having 1 to 10 carbon atoms; provided that each of the aryl groups and the heterocyclic groups for R¹ to R⁴ can have one or more substituents selected from the group consisting of an alkyl group having 1 to 6 carbon atoms, hydroxyl, amino, an alkylamino group having 1 to 6 carbon atoms, a dialkylamino group having 2 to 12 carbon atoms in total, an alkoxy group having 1 to 6 carbon atoms, a halogen atom, a haloalkyl group having 1 to 6 carbon atoms, cyano, nitro, carboxyl, an alkoxycarbonyl group having 2 to 7 carbon atoms, carbamoyl, an alkylaminocarbonyl group having 2 to 7 carbon atoms, a dialkylaminocarbonyl group having 3 to 13 carbon atoms in total, amidino, and guanidino.

7. A medicament for treatment or prophylaxis of cerebral aneurysms, wherein the medicament contains an epoxysuccinamide derivative having the formula (1) or its physiologically acceptable salt as an active ingredient: 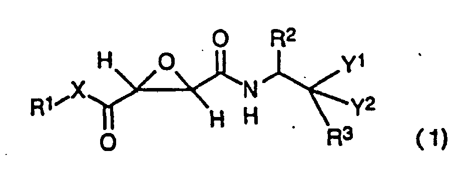 wherein R¹ represents a hydrogen atom, an alkyl group having 1 to 10 carbon atoms, an alkenyl group having 2 to 10 carbon atoms, an alkynyl group having 2 to 10 carbon atoms, or an aryl group having 6 to 20 carbon atoms, an aralkyl group comprising an aryl group having 6 to 20 carbon atoms and an alkyl group having 1 to 6 carbon atoms; R² represents isobutyl or isopropyl; R³ represents a hydrogen atom or an aryl group having 6 to 20 carbon atoms; X represents -O- or -NR⁴- in which R⁴ is a hydrogen atom, an alkyl group having 1 to 10 carbon atoms, an aryl group having 6 to 20 carbon atoms, an aralkyl group comprising an aryl group having 6 to 20 carbon atoms and an alkyl group having 1 to 6 carbon atoms, a heterocyclic
group having 3 to 12 carbon atoms, or a heterocyclicalkyl group comprising a heterocyclic group having 3 to 12 carbon atoms and an alkyl group having 1 to 6 carbon atoms; Y¹ represents OR⁵ in which R⁵ is a hydrogen atom, an alkyl group having 1 to 10 carbon atoms, an aryl group having 6 to 20 carbon atoms, an aralkyl group comprising an aryl group having 6 to 20 carbon atoms and an alkyl group having 1 to 6 carbon atoms, acetyl, benzoyl, a heterocyclic group having 3 to 12 carbon atoms, or a heterocyclic-alkyl group comprising a heterocyclic group having 3 to 12 carbon atoms and an alkyl group having 1 to 6 carbon atoms; Y² represents a hydrogen atom; provided that the alkyl group for R⁵ can have one or more substituents selected from the group consisting of hydroxyl, amino, an alkylamino group having 1 to 6 carbon atoms, a dialkylamino group having 2 to 12 carbon atoms in total, an alkoxy group having 1 to 6 carbon atoms, carboxyl, an alkoxycarbonyl group having 2 to 7 carbon atoms, -CONH₂, an alkylaminocarbonyl group having 2 to 7 carbon atoms, a dialkylaminocarbonyl group having 3 to 13 carbon atoms in total, and guanidino; each of the aryl groups for R¹, R³ and R⁵ can have one or more substituents selected from the group consisting of an alkyl group having 1 to 6 carbon atoms, hydroxyl, amino, an alkylamino group having 1 to 6 carbon atoms, a dialkylamino group having 2 to 12 carbon atoms in total, an alkoxy group having 1 to 6 carbon atoms, a halogen atom, a haloalkyl group having 1 to 6 carbon atoms, cyano, nitro, carboxyl, an alkoxycarbonyl group having 2 to 7 carbon atoms, -CONH₂, an alkylaminocarbonyl group having 2 to 7 carbon atoms, a dialkylaminocarbonyl group having 3 to 13 carbon atoms in total, amidino, and guanidine; the heterocyclic group for R⁵ can have one or more substituents selected from the group consisting of an alkyl group having 1 to 6 carbon atoms, hydroxyl, amino, an alkylamino group having 1 to 6 carbon atoms, a dialkylamino group having 2 to 12 carbon atoms in total, an alkoxy group having 1 to 6 carbon atoms, a halogen atom, a haloalkyl group having 1 to 6 carbon atoms, cyano, nitro, carboxyl, an alkoxycarbonyl group having 2 to 7 carbon atoms, -CONH₂, an alkylaminocarbonyl group having 2 to 7 carbon atoms, a dialkylaminocarbonyl group having 3 to 13 carbon atoms in total, amidino, and guanidine; and each of the heterocyclic groups for R⁴ and R⁵ is selected from the group consisting of pyridyl, pyrrolidinyl, piperidinyl, furyl, thienyl, piperazinyl, indolyl, and benzimidazolyl.

8. The medicament for treatment or prophylaxis as defined in any one of claims 5 to 7, wherein R¹ is a hydrogen atom or an alkyl group having 1 to 6 carbon atoms.

9. The medicament for treatment or prophylaxis as defined in claim 5 or 6, wherein R² is an alkyl group having 1 to 6 carbon atoms, phenyl, or benzyl.

10. The medicament for treatment or prophylaxis as defined in claim 5 or 6, wherein R³ is a hydrogen atom or an aryl group having 6 to 20 carbon atoms.

11. The medicament for treatment or prophylaxis as defined in any one of claims 5 to 7, wherein X is -O-.

12. The medicament for treatment or prophylaxis as defined in claim 5, wherein Y¹ is hydroxyl, an alkoxy group having 1 to 6 carbon atoms, acetoxy, or an aralkyloxy group comprising an aryl group having 6 to 20 carbon atoms and an alkyl group having 1 to 6 carbon atoms.

13. The medicament for treatment or prophylaxis as defined in claim 5, wherein R² is isobutyl or isopropyl, R³ is a hydrogen atom, Y¹ is OR⁵ whose R⁵ has the meaning defined in claim 5, and Y² is a hydrogen atom.

14. The medicament for treatment or prophylaxis as defined in claim 5, wherein R² is isobutyl or isopropyl, R³ is an aryl group having 6 to 20 carbon atoms, Y¹ is OR⁵ whose R⁵ has the meaning defined in claim 5, and Y² is a hydrogen atom.

15. The medicament for treatment or prophylaxis as defined in claim 5, wherein Y¹ and Y² in combination with each other forms =O.

16. The medicament for treatment or prophylaxis as defined in any one of claims 5 to 15, wherein the physiologically acceptable salt is an alkali metal salt.

17. A medicament for treatment or prophylaxis of cerebral aneurysms, wherein the medicament contains an epoxysuccinamide derivative having the following formula or its physiologically acceptable salt as an active ingredient.
